# EUROPEAN PATENT APPLICATION

(11) **EP 4 748 415 A1**
(43) Date of publication of application: **27.05.2026**
(21) Application number: 24214208.1
(22) Date of filing: 20.11.2024
(51) Int. Cl.: A61M 5/315

(54) **DOSING BUTTON ASSEMBLY FOR AN INJECTION DEVICE**

(71) Applicant: Ypsomed AG, 3401 Burgdorf (CH)
(72) Inventor: Kalbermatter, Gabriel, 3400 Burgdorf (CH); Bosshard, Simon Martin, 3324 Hindelbank (CH); Schrul, Christian, 3414 Oberburg (CH); Hugentobler, Simon, 3097 Liebefeld (CH); Koenig, Marc, 3512 Walkringen (CH)

(57) **Abstract**

Disclosed is a dose button assembly (1, 3) for an injection device (8) that enables reliable transmission of the injection force to a dosing member (2, 4) of the device and comprises an overload protection mechanism. It comprises a button clutch element (3) and a button housing (1), is releasably engageable with a first part (2) of a dosing member of the injection device (8) and is rotatably mounted on a second part (4) of the dosing member of the injection device (8).

## Description

### FIELD OF THE INVENTION

The present invention relates to the field of medicament delivery devices, in particular injection devices. More precisely, the invention relates to a knob or button assembly for an injection device, used to dial a dose and trigger an injection.

### BACKGROUND OF THE INVENTION

Injection devices with variable dosing, such as injection pens for the delivery of insulin often comprise a screw-type drive mechanism with a force/stroke conversion wherein a user can dial a dose by screwing a dosing member or dial/dose sleeve out of the pen. To effectuate injection, the user can push/screw the dosing member back into the pen, performing an injection stroke, wherein the dosing member rotates during said injection stroke. The relatively small force applied by the user is converted via threads or gears into a short high force stroke of a plunger rod that drives a medicament out of a container held in the device. Examples of such devices are described in WO10072427 Al or WO9938554 A1. Said dosing member usually comprises a push button or dosing button, rotatably mounted to the sleeve, which comprises a push surface for the user and does not rotate with the sleeve during the injection stroke but rotates with the sleeve for setting a dose. The torque-transmitting connection between the button needs to be disengaged during injection and the push force applied by the user thus needs to be converted to a rotation via a pivot bearing or a rotation interface between the dosing member and the dosing button. To this end, a variety of button designs and ways of connection between the dosing member and the button have been developed.

US9265893B2 discloses a connection between a driving part and a button of an injection device comprising a central pivot bearing and radial bearings that stabilize the button laterally. Friction between the button and the driving part is minimized by a raised pointer forming a pivot and minimizing the contact surface.

A further concern for the construction of such devices is overloading of the drive or dosing mechanism by applying excessive torque. Many injection pens, especially disposable ones, are produced from plastic which is cheap and fast to manufacture but has limited tensile strength. Thus, the mechanism of the pen can be susceptible to breaking if the user applies a force or torque which exceeds a certain value. Different overload protection solutions are known from the prior art.

WO2011057677A1 discloses a drug administering device comprising a clutch with an overload protection mechanism between the drive mechanism and the dosing/injection button. The clutch comprises an input and an output member, wherein torque from the injection button is only propagated to the drive mechanism up to a threshold value.

WO2015/101669A1 discloses an injection pen with a torque limiting mechanism comprising a flexible portion on a reset tube of the device. Over a certain torque limit, the flexible portion can bend radially inwards, and the torque is no longer propagated from the dosing button to the reset tube, protecting the drive mechanism of the pen from overload.

Many of the prior art solutions for dosing knobs or buttons are complicated and require extra parts for overload protection. In addition, the pivot bearing between the button and the pen mechanism in the prior art may be unsuitable for reusable devices, as the contact surfaces between the parts is small and thus susceptible to wear.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances

The term "distal" is meant to refer to the direction or the end of the drug delivery device carrying an injection needle or an injection cannula, whereas the term "proximal" is meant to refer to the opposite direction or end pointing away from the needle or cannula.

The term "injection system" or "injector" refers to a device that is removed from the injection site after each medication event or drug delivery process, whereas the term "infusion system" refers to a device with a cannula or needle that remains in the skin of the patient for a prolonged period of time, for example, several hours.

### DESCRIPTION OF THE INVENTION

It is an objective of the invention to provide a dosing button (hereafter referred to as "button") assembly for an injection device, which button assembly is optimized in view of friction and force transmission.

This objective is achieved by the button assembly as described in the independent claim. Possible refinements and/or improvements are detailed in the dependent claims. The assembly comprises a button housing and a button clutch element, wherein the button housing can be gripped and rotated by the user to dial a dose and wherein the button housing is rotationally coupled to the button clutch element. The button clutch element releasably connects the button housing rotationally to the dosing member of the injection device and is switchable between an engaged and a disengaged state, wherein in the engaged state, during dose setting and correcting, the clutch element is in torque-transmitting engagement with the dosing member and in the disengaged state, during dispensing, the clutch element is rotationally decoupled from the dosing member and the dosing member can rotate about a longitudinal axis (L) of the injection device relative to the button assembly. The longitudinal axis (L) extends along the longitudinal axis of the injection device housing and corresponds to a cross-sectional center of the injection device.

The button assembly is rotatably mounted to a proximal portion of the injection device, more precisely to a dosing member or dosing sleeve, which is part of the dosing mechanism of the device. The button housing is preferably sleeve-shaped and the radially outwards facing side wall of the button housing is adapted to be gripped and rotated by a user to dial or set a dose to be administered by the injection device. The button housing preferably comprises a proximal end wall which, in a mounted state, forms a proximal end of the injection device and comprises as a push surface, whereon the user can apply a distally oriented force to effectuate medicament dispensing after a dose has been dialed. Preferably, the button housing has the same or a similar cross-section as the injection device housing. In a preferred embodiment, the cross section is circular. The button housing is axially and rotationally fixed or coupled to the button clutch element.

The button clutch element is releasably rotationally coupled to the dosing member and can be rotationally decoupled by an axial decoupling movement or decoupling stroke, preferably in distal direction. The clutch is thus switchable between an engaged and a disengaged state, wherein the clutch element is in an engaged or coupled state during dose dialing and in a disengaged or decoupled state during medicament dispensing, in which the dosing member can rotate relative to the button clutch element and the button housing. A pivot bearing is formed between the button clutch element and the dosing member. The button clutch element is preferably disc-shaped and comprises a stem part that extends from a center of the clutch element along the longitudinal axis (L) in distal direction.

The stem part has a cylindrical shape with a radial dimension that is smaller, preferably at most half of that of the disc-shaped part of the button clutch element and is rotationally symmetric about the longitudinal axis (L). The axial length of the stem part is at least one third of the axial length or height of the button housing. In a preferred embodiment, the stem part extends into a bore of the dosing member, wherein the bore comprises a distal bottom surface that is in contact with a distal end surface of the clutch element stem part and forms a pivot bearing on which the clutch element can rotate relative to the dosing mechanism. In a preferred embodiment, the stem part is in contact with the lateral side walls of the bore over the entire length of the bore, such that the clutch element is securely and stably mounted on the dosing mechanism. To minimize friction between the bore and the stem part, the two parts are ideally made from materials with low relative coefficients of friction, such as polyoxymethylene (POM) and polycarbonate (PC) plastics.

The button clutch element can engage the dosing member for rotational, torque-transmitting coupling of the button clutch element to the dosing member. The engagement may be by positive fit, friction fit or any other form or releasable engagement.

The mounting of the button clutch element to the dosing member via a stem part, that is surrounded and in contact with side walls of a bore in the dosing member provides for a stable coupling of the button to the dosing member, which is suitable for repeated use and resistant to wear.

In a preferred embodiment, the torque-transmitting engagement between the button clutch element and the dosing member is achieved by a set of teeth on the button clutch element that can releasably engage a set of corresponding teeth on the driving mechanism. This allows for a compact design of the button assembly, while maintaining a highly efficient transmission of the torque applied by the user.

Alternatively, the button clutch element and the dosing member may be engaged by any other form fit or force fit or by a contactless means such as magnets.

The teeth on the button clutch element are preferably pointed or directed radially inwards, towards the longitudinal axis (L), wherein the tips extend at least approximately parallel to the longitudinal axis (L). The tip of a tooth is to be understood as the outer or innermost edge where the two torque or force transmitting surfaces or flanks of the tooth meet. The teeth are arranged on the periphery of the button clutch element. In a preferred embodiment, multiple toothed fingers extend in the distal direction from a disc-shaped main portion of the button clutch element. Preferably, the fingers do not extend beyond the distal end of the button housing. The fingers comprise protrusions or teeth that can releasably engage counterpart protrusions or teeth on the dosing mechanism such that torque applied by the user to the button housing is propagated from the housing to the clutch element and further to the dosing mechanism.

In a further preferred embodiment, the teeth on the dosing member comprise tips which do not extend parallel to the longitudinal axis but extend along lines that are angled with respect to the longitudinal axis. More precisely, the tips extend along a set of lines that lie on the surface of a circular cone with a tip that lies on the central longitudinal axis (L), proximally of the teeth. The angle is chosen in a way that the tips of the teeth of the dosing member extend parallel to the teeth of the clutch element, or to the teeth on a flexible member of the clutch element when said flexible members are flexed outwards. This has the advantage of reducing wear and plastic deformation on the teeth of the dosing member, as the teeth on the flexible members of the clutch element do not only rattle over a proximal corner of the teeth of the dosing member but instead the contact area is extended over the entire length of the teeth and the force is more evenly distributed.

The button clutch element teeth are engaged and axially aligned with the teeth on the dose setting member in a dose setting stage, when the user rotates the button housing, such that the applied torque is propagated to the dosing member. During the dose setting operation the dose setting member, together with the button assembly is screwed out of a proximal end of an injection device housing. When a desired dose has been set by the user, a distally directed force may be applied by the user to the proximal push surface of the button housing, upon which the button assembly is shifted by a decoupling or disengaging stroke along the longitudinal axis (L), relative to the dose setting member or to a part of the dose setting member, such that the teeth of the button clutch element and the teeth on the dose setting member are no longer axially aligned and the button clutch element is in the disengaged or decoupled state, in which the dosing member is free to rotate relative to the button clutch element. After dispensing, the button clutch element may be returned to the initial position by an elastic member such as a compression spring.

The button assembly additionally comprises an overload protection mechanism which is adapted to protect the dosing mechanism and/or the dosing member from damage resulting from excessive torque applied to the button or the button housing by the user. In the dose setting stage, the teeth of the button clutch element stay engaged with the teeth on the dose setting member until a threshold torque is reached, upon which the teeth can radially disengage. The teeth may be arranged on or extend from a flexible or resilient element affixed to the button clutch element. Alternatively, the button clutch element may be made from a flexible material and the teeth might directly extend from the body of the clutch element itself, wherein the clutch element can elastically deform upon reaching the threshold torque. In a preferred embodiment, the button clutch element teeth, which are located on distally extending fingers extending from the button clutch element periphery, the fingers can radially flex outwards in a space between the button housing and the button clutch element. If the user continues to apply a torque above the threshold, the fingers can repeatedly flex outwards and the clutch element teeth can rattle over the teeth on the dosing member.

The button housing and the button clutch element are rotationally and axially couped or fixed. The coupling may be achieved by an adhesive connection, a positive fit, a snap-fit connection or any other suitable means. In a preferred embodiment the axial coupling is achieved by an annular snap-fit connection for the axial coupling and a positive fit between protrusions on the button clutch element and recesses on the button housing for the rotational coupling. Preferably, a circumferential, annular protrusion around the periphery of the button clutch element engages an annular recess on the inner periphery of the button housing, forming an annular snap-fit connections and axially coupling the button clutch element to the button housing. Further preferably, the rotational coupling is achieved by rectangular protrusions, extending from an inner periphery of the button housing along the longitudinal axis (L) and engaging corresponding protrusions on the outer periphery of the button clutch element. The stem part of the clutch element forms a distal end of the button clutch element. Preferably the pivot bearing between the dosing member and the button clutch element is formed on a distal end of the clutch element, more preferably on a distal end of the stem part of the clutch element.

The teeth of the button clutch element engage a first part of the dosing member, and the pivot bearing is formed between the button clutch element, and a second part of the dosing member. The first and the second part of the dosing member are preferably independently produced and are releasably assembled in a final assembly step. Ideally, different materials or plastics are chosen such that the relative coefficient of friction is low, and that relative motion is possible even if the parts are in contact. The necessity for this will become apparent later. The first part and the second part of the dosing member make up the dosing member of the injection device, wherein the dosing member is part of the dosing and dispensing mechanism of the injection device.

The clutch element is moveable along the longitudinal axis (L) relative to the first part of the dosing member and is engageable and disengageable with said first part of the dosing member by an engaging stroke along the longitudinal axis (L).

In a preferred embodiment the first part of the dosing member is sleeve-shaped and concentrically extends along the longitudinal axis (L). Preferably, the teeth on the first part of the dosing member are located on at least two arms that extend from a proximal end of the first part of the dosing member and are arrange evenly or equidistantly around the circumference of the first part of the dosing member. In a preferred embodiment the number of arms is two. The first part of the dosing member additionally comprises a thread segment which is adapted to engage a thread, preferably an outer thread, on a part of the housing or housing insert or the dosing mechanism of the injection device. When the user dials or dispenses a dose the first part of the dosing member is screwed along said thread in the proximal (dosing) or the distal (dispensing) direction respectively.

In a preferred embodiment, the second part of the dosing member is sleeve-shaped and concentrically extends along the longitudinal axis (L). It is preferably arranged radially inside the first part of the dosing member. It comprises a thread on an outer surface which is adapted to be engaged with a thread or a thread segment of the housing of the injection device or the dosing mechanism of the injection device. When the user dials or dispenses a dose the second part of the dosing member is screwed along the thread or a thread segment of the housing of the injection device or the dosing mechanism of the injection device in the proximal (dosing) or the distal (dispensing) direction respectively.

The first part of the dosing member preferably comprises a sequence of numbers or markings corresponding to dose units dispensable by the injection device. The numbers, or more precisely one number or marking may be visible through a window or opening in the injection device housing, such that the user can visually confirm what dose is being dialed at any given time. The numbers or markings may be arranged helically along the length or along a part of the length of an outer surface of the first dosing member. The pitch of the helix preferably corresponds to the pitch of the inner thread segment on the first part of the dosing member.

The second part of the dosing member is moveable along the longitudinal axis (L), relative to the first part of the dosing member. After a dose has been dialed, the user applies a distal force on the push surface of the button housing, upon which the button clutch element, together with the second part of the dosing member, undergoes an axial disengagement or decoupling stroke, relative to the first part of the dosing member. The disengagement or decoupling stroke is at least equivalent to an axial dimension of the teeth on the button clutch element.

The first and the second part of the dosing member are preferably permanently rotationally coupled, such that no relative rotation between the first and the second part of the dosing member is possible. In a preferred embodiment the outer surface or wall of the second part of the dosing member is in contact with an inner surface or wall of the first part of the dosing member over a portion of the length of the second part of the dosing member, wherein the inner and outer wall surface or wall have non-circular cross-sections along the longitudinal axis (L), such that relative rotation is made impossible. The button clutch element is arranged within the button housing. More preferably, the button clutch element is completely contained within the button housing. This provides a compact design.

The assembly as described above is preferably part of an injection device used to self-administer medication or therapeutic substances. The injection device is preferably handheld and pen-shaped and is adapted to hold a medicament container such as a cartridge or a pre-filled syringe containing the medication or therapeutic formulation. The medicament container may be replaceable, and the device may be reusable with a new cartridge or syringe, or the device may be disposed after the contents of the container have been dispensed. In case of a reusable device, the device may comprise a cartridge or container holder which is releasably attachable to a main part containing the mechanism for dosing and dispensing.

### BRIEF DESCRIPTION OF THE DRAWINGS

The subject matter of the invention will be explained in more detail in the following text with reference to preferred exemplary embodiments which are illustrated in the attached drawings, in which:
- Figure 1: depicts am injection pen comprising a button assembly according to the present invention
- Figure 2: depicts a perspective view of the button assembly connected to the first part of the dosing member
- Figure 3: depicts a perspective view of the second button part connected to the first part of the dosing member without button housing
- Figure 4: depicts a perspective view of the second button part removed from the first part of the dosing member
- Figure 5: depicts a section view of the button assembly, connected to the first and second part of the dosing member, in a dosing state
- Figure 6: depicts a section view of the button assembly, connected to the first and second part of the dosing member, in a dispensing state
- Figure7a: depicts a second version of the first part of the dosing member removed from the button clutch element
- Figure: 7bdepicts a close-up perspective view of the button clutch element engaged to the second version of the first part of the dosing member

The reference symbols used in the drawings, and their primary meanings, are listed in summary form in the list of designations. In principle, identical parts are provided with the same reference symbols in the figures.

### DETAILED DESCRIPTION OF PREFERRED EMBODIMENT

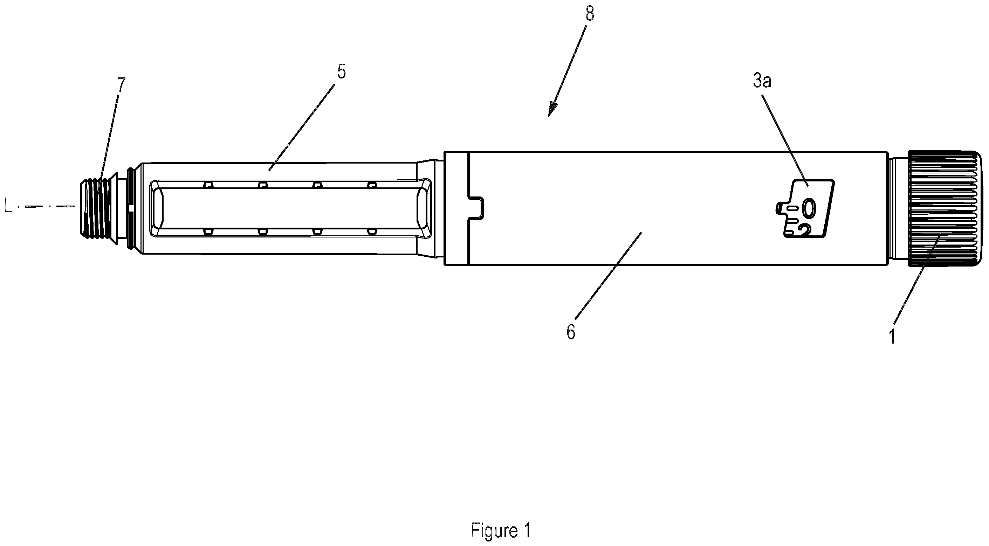

Figure 1 depicts an injection device 8 comprising a button assembly according to the present invention, wherein only the button housing 1 is visible. Button housing 1 is, during the dose setting stage, rotationally coupled to the first part of the dosing member 3a, which is visible through an opening in the housing 6 of the injection device 8. Distally of the main housing 6, cartridge holder 5 is attached thereto. Cartridge holder 5 may be releasably attached, such that it can be removed from the main housing 6 and the contained cartridge can be replaced, or it may be permanently attached, such that the entire pen is to be disposed after dispensing the contents of the cartridge. A needle thread 7 allows for attachment of a disposable needle, which must be replaced after each use.

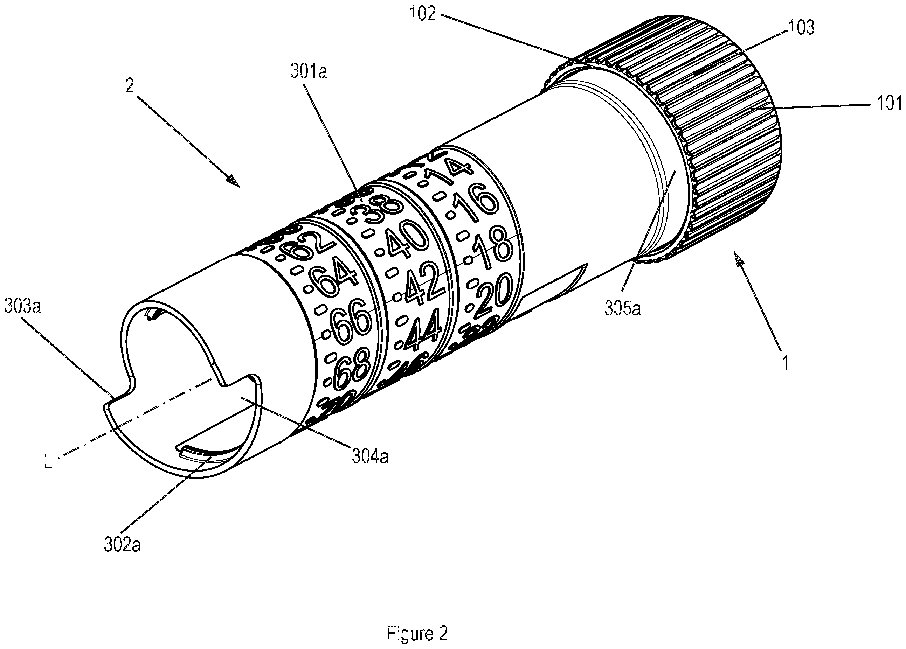

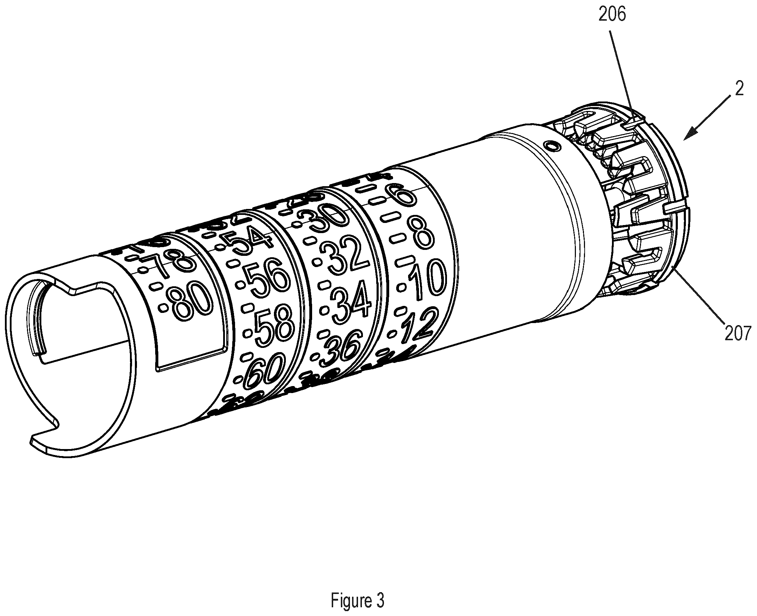

In Figure 2, a perspective view of button housing 1, mounted to the first part 3a of the dosing member is shown. The first part of the dosing member may also be referred to as scale drum. Button housing 1 has a cylindrical shape and comprises side walls 103 with a circular cross-section around a central longitudinal axis (L) and a proximal end wall that forms a push surface for the user (not visible). Scale drum 3a is generally sleeve shaped with two triangular recesses 304a at a distal end that define two radial stop surfaces 303a. The proximal end comprises a radially extended section 305a (as shown in figure 3) of increased thickness that forms a positive fit with - or closely follows the inner periphery 102 of the button housing to prevent dirt or dust from entering. It comprises a dose scale 301a in the form of a helical sequence of numbers corresponding to dose units dispensable from the injection device. The dose scale can be an integral part of the scale drum or can be printed or engraved or etched thereon. The assembled injection device comprises a viewing window in an outer housing (not shown) giving visible access to part of the dose scale, preferably to a single number of the dose scale, such that the user can see what dose is dialed at any given moment. As will be described in more detail later, button housing 1 is not rotatable relative to scale drum in the shown position but is rotationally coupled thereto. Button housing 1 comprises gripping grooves 101 on the outer surface to improve grip. To dial a dose, the user holds the button housing 1 and rotates clockwise for increasing and counterclockwise for decreasing the dose, wherein scale drum 3a rotates with the button housing. Due to thread segments 302a, which are in engagement with a thread on an inner housing part (not shown), the button, together with the scale drum, undergoes a screwing motion and is axially displaced in a proximal direction.

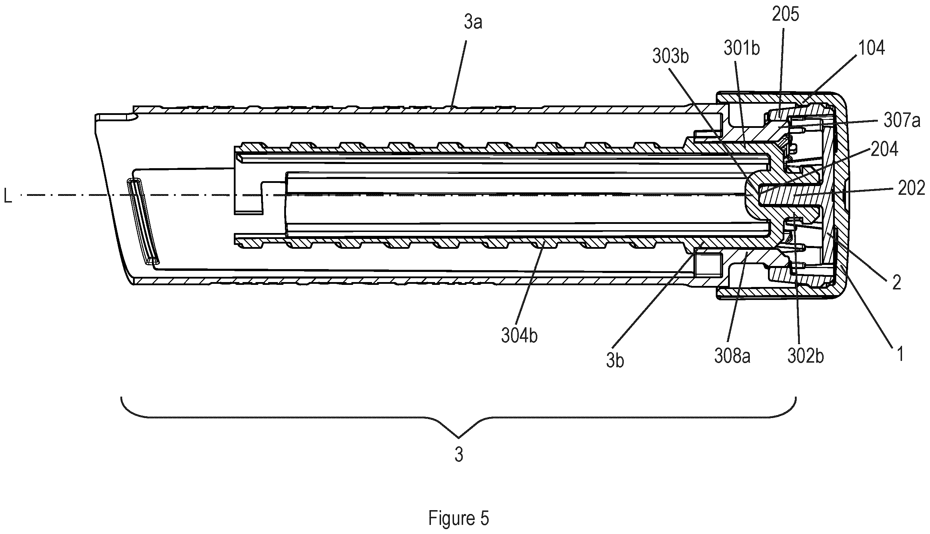

Figure 3 depicts a perspective view of the button clutch element 2, attached to the scale drum 3a, wherein the button housing 1 has been removed. Button clutch element 2 is rotationally secured to button housing 1 by a set of recesses 206 arranged around the periphery and adapted to engage corresponding protrusions on the inner periphery of button housing 1. In addition, button clutch element 2 is axially secured to the button housing 1 by a circumferential protrusion or snap ring 207, adapted to form a non-releasable annular snap-fit connection by snapping behind corresponding protrusions (104, shown in figure 5) on an inner proximal part of the button housing side walls 103.

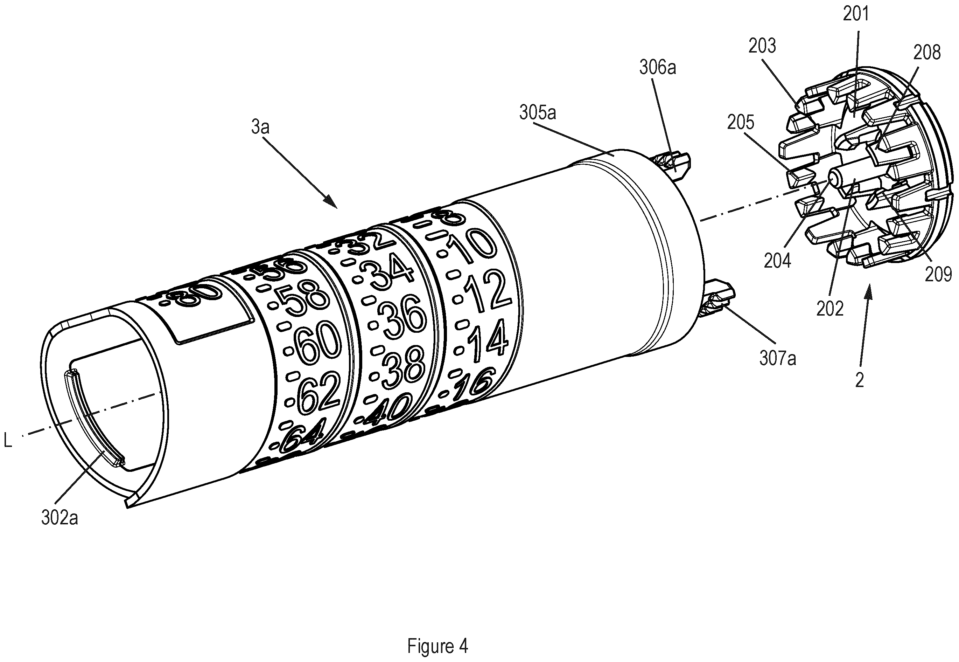

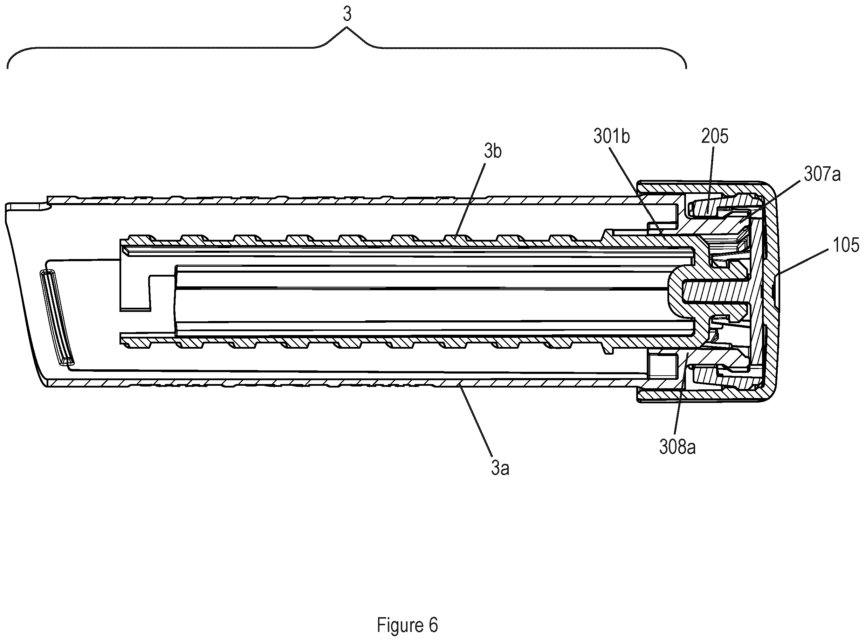

Figure 4 is a perspective view of scale drum 3a with removed button clutch element 2. Two toothed arms 306a extend from a proximal end of the scale drum and comprise a set of teeth 307a which are adapted to engage the teeth 205 of the button clutch element 2. Arms 306a are offset around the longitudinal axis (L) by 90° with respect to the thread segments 302a, which allows for a simplified injection molding process, as the part can be pushed out of the mold during demolding instead of being screwed out. Button clutch element 2 comprises a disc-shaped main part 201 and a stem part 202 which extends from the center of the disc shaped part 201 in the distal direction along the longitudinal axis (L). The distal end surface 204 of the stem forms a pivot bearing with a second part of the dosing member (not shown). The teeth 205 of the button clutch element are arranged on arms 203 extending in distal direction from the periphery of disc-shaped main part of the button clutch element 2. Four snap fingers 208 are arranged concentrically around the stem part 202 and comprise protrusions 209, which are adapted to form a non-releasable snap connection with a second part of the dosing member, concentrically arranged within the scale drum (shown in Figure 5 and 6).

Figure 5 shows a section view of the button assembly 1, 2 connected to the scale drum 3a and the second part of the dosing member 3b. The second part of the dosing member may also be referred to as drive sleeve. The injection device is in a dose selecting state, with the button assembly 1, 2 in its proximal position. Teeth 205 of the button clutch element are engaged with teeth 307a of the scale drum in a torque transmitting positive fit. The stem part 202 of the button clutch element 2 extends into a bore 302b of the drive sleeve 3b. A pivot bearing is formed between the distal end surface of the stem part 204 and the bottom 303b of bore 302b. To dial a dose the user holds button housing 1 and rotates clockwise. The torque is propagated to button clutch element 2 via protrusions on the button housing 1 engaged in the recesses 206 (figure 2). Scale drum 3a is rotationally locked to dose sleeve 3b via the inwards facing walls 308a of arms 306a that are in contact with a planar wall 301b of the dose sleeve 3b, the planar wall being formed by a cutout that has the shape of a disk segment when viewed along the longitudinal axis (L). Drive sleeve 3b comprises an outer thread 304b which is engaged with an inner thread on a part of the housing (not shown).

The button assembly 1, 2 additionally comprises an overload protection feature, which protects the mechanism from damage if too much torque is applied by the user. This can for instance be the case if the maximum dose is dialed, and the user still tries to increase the dose or when no dose is dialed, and the user still tries to decrease the dose. Other causes can be an occlusion or an obstruction in the syringe. If the torque applied by the user is exceeding a threshold value, the toothed arms 203 (figure 3) can radially flex outwards and the teeth 205 of button clutch element 2 get out of engagement with teeth 307a of scale drum 3a.

Figure 6 shows a section view of the button assembly 1, 2 connected to the scale drum 3a and the drive sleeve 3b in a dose dispensing state with the button assembly in its distal position when the user applies a distally oriented force to push surface 105 of button housing 1. Drive sleeve 3b, button clutch element 2 and button housing 1 are shifted in distal direction relative to scale drum 3a. Teeth 307a of the scale drum are no longer engaged with teeth 205 of the button clutch element, allowing scale drum 3a and dose sleeve 3b, which are still rotationally coupled via inwards facing surface of arms 308a and planar surface 301b, to rotate relative to the button assembly 1, 2 while the entire arrangement moves in distal direction during dispensing.

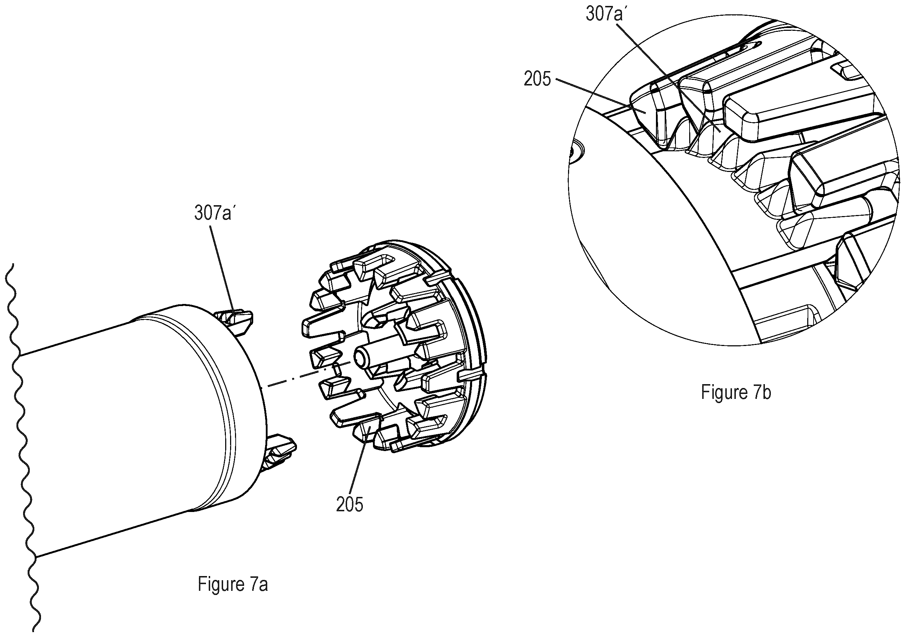

Figure 7a shows a perspective view of the button clutch element removed from a second version of the first part of the dosing member (scale drum). In this version the edges or tips of teeth 307a' of the scale drum do not extend parallel to the longitudinal axis (L) but are angled, or extend along a line which is non-parallel to the longitudinal axis (L). In particular, a distal end of the tips comb or ridge of the teeth is radially shifted outwards, such that the lines along the tips are contained in a lateral surface of a cone with a tip that is on the longitudinal axis, proximally of the button assembly. This has the advantage that, when teeth 205 are flexed outwards, due to excessive torque application by the user, the entire length of the tips of teeth 205 is in contact with teeth 307a', such that wear is reduced compared to the first version of the scale drum wherein in an outwards flexed state of teeth 205, only a point on both teeth 205, 307a'is in contact. Figure 7b depicts a perspective view of the button clutch element engaged with the second version of the first part of the dosing member.

While the invention has been described in detail in the drawings and foregoing description, such description is to be considered illustrative or exemplary and not restrictive. Variations to the disclosed embodiments can be understood and effected by those skilled in the art and practicing the claimed invention, from a study of the drawings, the disclosure, and the appended claims. In the claims, the word "comprising" does not exclude other elements or steps, and the indefinite article "a" or "an" does not exclude a plurality. The mere fact that certain elements or steps are recited in distinct claims does not indicate that a combination of these elements or steps cannot be used to advantage, specifically, in addition to the actual claim dependency, any further meaningful claim combination shall be considered disclosed.

### LIST OF DESIGNATIONS

- 1: Button Housing
- 2: Button Clutch Element
- 3: Dosing Member
- 3a: Scale Drum
- 3b: Drive Sleeve
- 5: Cartridge Holder
- 6: Injection Device Housing
- 7: Needle Thread
- 8: Injection Device
- 101: Griping Grooves
- 102: Inner Periphery of Button Housing
- 103: Side Walls of Button Housing
- 104: Protrusions
- 201: Button Clutch Element Disc Part
- 202: Button Clutch Element Stem Part
- 203: Toothed Arms
- 204: Stem Part Distal End Surface
- 205: Button Clutch Element Teeth
- 206: Recess
- 207: Snap Ring
- 208: Snap Fingers
- 209: Protrusions
- 301a: Dose Scale
- 302a: Thread Segment
- 303a: Stop Surface
- 304a: Triangular Recess
- 305a: Radially Extended Section
- 306a: Toothed Arms
- 307a: Teeth
- 307a': Angled Teeth
- 308a: Inwards Facing Wall
- 301b: Planar Wall
- 302b: Bore
- 303b: Bottom of Bore
- 304b: Thread

## Claims

1. A rotatable dose button assembly for an injection device, the assembly comprising a button housing (1) and a button clutch element (2), for releasably coupling the button housing (1) to a dosing member (3) of the injection device, wherein
o the button housing is adapted to be gripped and rotated by a user and is rotationally coupled to the button clutch element,
o the button clutch element is switchable between an engaged state and a disengaged state, wherein in the engaged state, the dosing member rotates with the button clutch element during dose setting and correction and in a disengaged state, during dispensing, the dosing member can rotate relative to the button clutch element about the longitudinal axis (L) of the injection device,
**characterized in that**,
the button clutch element additionally comprises a stem part (202) extending along the longitudinal axis, which stem part is adapted to be rotatably mounted on the dosing member of the injection device via a pivot bearing.

2. Assembly according to claim 1, wherein the engagement between the button clutch element (2) and the dosing member (3) is achieved by a set of teeth (205) on the button clutch element that engage a set of corresponding teeth (307a) on the dosing member.

3. Assembly according to claim 2, wherein the teeth on the button clutch element (205) are directed radially inwards, such that the tips of the teeth point towards the longitudinal axis (L).

4. Assembly according to claim 2 or 3, wherein the teeth on the dosing member (307a) are directed radially outwards and the tips of the teeth extend along lines that are angled relative to the longitudinal axis.

5. Assembly according to any of claims 2 to 4, wherein the teeth on the button clutch element (205) are engaged with the teeth on the dosing member (307a) in a dose setting stage of the injection device and are disengaged from the teeth on the dosing member in a dispensing stage of the injection device.

6. Assembly according to any of claims 2 to 5, wherein in the dose setting stage, the teeth on the button clutch element (205) are adapted to stay engaged with the teeth on the dosing member up to a threshold torque applied by the user and wherein, upon reaching said threshold torque, the teeth can radially disengage from the teeth on the dosing member.

7. Assembly according to any of claims 1 to 6, wherein the connection between the button housing (1) and the button clutch element (2) is a snap-fit connection.

8. Assembly according to any of claims 2 to 8, wherein the teeth (205) on the button clutch element can be engaged with teeth (307a) on a fist part (3a) of the dosing member and the pivot bearing is formed with a second part (3b) of the dosing member.

9. Assembly according to claim 9, wherein the button clutch element (2) is moveable along the longitudinal axis (L) relative to the first part of the dosing member (3a) and is engaged by an engaging stroke along the longitudinal axis.

10. Assembly according to claim 9, wherein the first part (3a) of the dosing member is sleeve-shaped and comprises a thread segment (302a) on an inner surface, adapted to be in a threaded engagement with a thread of a housing of the injection device.

11. Assembly according to claim 9 or 10, wherein the second part (3b) of the dosing member is sleeve-shaped and comprises a thread (304b) on an outer surface adapted to be in a threaded engagement with the housing

12. Assembly according to any of claims 9 to 11, wherein the first part of the dosing member comprises a helically arranged sequence of numbers or markings (301a).

13. Assembly according to any of claims 9 to 12, wherein the first part (3a) of the dosing member is moveable along the longitudinal axis (L) relative to the second part (3b) of the dosing member.

14. Assembly according to any of claims 9 to 13, wherein the first (3a) and the second (3b) part of the dosing member are rotationally coupled.

15. A handheld injection device (8) comprising a button assembly and a dosing and dispensing mechanism according to any of the preceding claims.
